# EUROPEAN PATENT SPECIFICATION

(11) **EP 1 416 931 B1**
(45) Date of publication and mention of the grant of the patent: **13.06.2007**
(21) Application number: 02764704.9
(22) Date of filing: 17.07.2002
(51) Int. Cl.: A61K 31/425, A61P 25/00, A61P 43/00

(54) **INHIBITORS OF POLYQ-AGGREGATION**
HEMMER DER AGGREGATION VON POLYQ
INHIBITEURS DE POLYQ-AGREGATION

(30) Priority: 13.08.2001 EP 01118838
(43) Date of publication of application: 12.05.2004
(73) Proprietor: MAX-PLANCK-GESELLSCHAFT ZUR FÖRDERUNG DER WISSENSCHAFTEN E.V., 80539 München (DE)
(72) Inventor: BÖTTCHER, Henning, 64287 Darmstadt (DE); HERHAUS, Christian, 64625 Bernsheim (DE); BARNICKEL, Gerhard, 64293 Darmstadt (DE); WANKER, Erich, E., 13187 Berlin (DE); HEISER, Volker, c/o Grohmann, 10999 Berlin (DE); LEHRACH, Hans, 14129 Berlin (DE); BROEKER, Wofgang, 15831 Mahlow (DE); DUNKEL, Ilona, 14195 Berlin (DE)
(74) Representative: Vossius & Partner
(86) International application number: PCT/EP2002/007912
(87) International publication number: WO 2003/015772

(56) References cited:
- EP-A- 0 282 971
- EP-A- 0 374 041
- EP-A- 0 507 318
- EP-A- 0 855 391
- WO-A-00/73282
- WO-A-99/65516
- US-A- 5 348 969
- US-A- 5 795 903
- PEREIRA, E R, ET AL.: "Syntheses and antimicrobial activities of five-membered ring heterocycles coupled to indole moieties" JOURNAL OF ANTIBIOTICS, vol. 49, no. 4, 1996, pages 380-385, XP009003375

## Description

The invention relates to the use of compounds of formula I 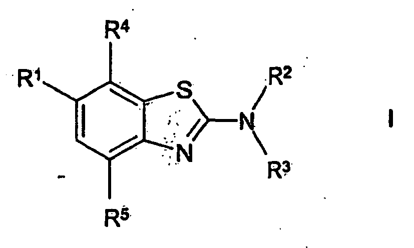 wherein
- R¹: is OH, OA or Hal
- R², R³: are independently of each-other H or A,
- R² and R³: together are an alkylene chain with 4, 5 or 6 C atoms,
- R⁴, R⁵: are independently of each other A or Hal,
- A: is alkyl with 1, 2, 3, 4, 5 or 6 C atoms,
- Hal: is F, Cl, Br or I,
and their salts, solvates and stereoisomers for the preparation of a pharmaceutical for inhibiting the formation of polyQ-aggregation.

Preferably, the invention relates to compounds selected from
2-amino-7-chloro-6-hydroxy-4-methyl-benzothiazole,
2-amino-4-chloro-6-hydroxy-7-methyl-benzothiazole,
2-amino-5,7-dimethyl-6-hydroxy-benzothiazole,
6-methoxy-4,7-dimethyl-benzothiazol-2-ylamine,
N-(6-methoxy-4,7-dimethyl-benzothiazol-2-yl)-N-methylamine
and their salts, solvates and stereoisomers.

Furthermore, the invention relates to the use of a compound selected from
2-amino-7-chloro-6-hydroxy-4-methyl-benzothiazole,
2-amino-4-chloro-6-hydroxy-7-methyl-benzothiazole,
2-amino-6-hydroxy-4-methyl-benzothiazole,
2-amino-5,7-dimethyl-6-hydroxy-benzothiazole,
2-dimethylamino-6-hydroxy-benzothiazole,
2-amino-4,7-dimethyl-6-hydroxy-benzothiazole,
6-methoxy-4,7-dimethyl-benzothiazol-2-ylamine,
N-(6-methoxy-4,7-dimethyl-benzothiazol-2-yl)-N-methylamine
and their salts, solvates and stereoisomers for the preparation of a pharmaceutical for inhibiting the formation of polyQ-aggregation.

The invention was based on the object of finding compounds having valuable properties, in particular those which can be used for the production of medicaments.

Surprisingly, it has been found that above-mentioned compounds and their salts, solvates and stereoisomers inhibit in vitro and in vivo formation of polyQ-aggregation. The accumulation of polyQ plays a direct role in the pathogenesis of neurodegenerative diseases (H.T.Orr, Development 15:925-932, 2001) such as Huntington's disease (V. Heiser et al., Proc. Natl. Acad. Sci. USA, 97, 6739-6744, 2000).

The compounds can be employed as pharmaceutical active compounds in human and veterinary medicine.

Other 2-amino-benzothiazole derivatives are described, for example, in EP 0 282 971 as cerebrovascular agents.

The following compounds are known:
2-amino-6-hydroxy-4-methyl-benzothiazole, synthesis is described by P.T.S. Lau and T.E. Gompf in J. Org. Chem. Vol. 35, 4103 - 4108;
2-dimethylamino-6-hydroxy-benzothiazole, CARN 943-04-4;
2-amino-4,7-dimethyl-6-hydroxy-benzothiazole, CARN 26278-83-1;
benzothiazole-2,5,6-triamine, CARN 313241-12-2;
[6,6']bibenzothiazolyl-2,2'-diamine, CARN 53357-04-3;
6,6'-thiodi(benzothiazole-2-amine), CARN 53357-07-6;
2,2'-m-phenylenedi(benzothiazole-6-amine), CARN 331653-50-0;
4-(6-methyl-benzooxazole-2-yl)-phenylamine, CARN 22501-77-5
2-(3-amino-phenyl)-quinoline-4-carboxylic acid, CARN 78660-91-0
2,7-dioxa-1,3,4,5,6,8,9,10-octaaza-dicyclopenta[a,e]cyclooctene, CARN 131122-64-0.
2,8,14,20-Tetrakis(2-chlorophenyl)-pentacyclo=[19.3.1.1^{3,7}.1^{9,13},1^{15,19}]octacosa-1(25),3,5,7(28),9,11,13(27),15,17,19(26),21,23-dodecaen-4,6,10,12,16,18,22,24-octol =

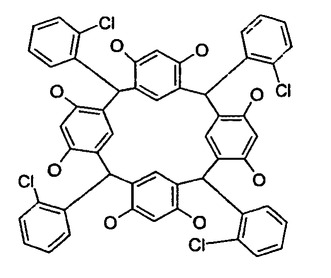

Furthermore, the invention relates to the use of a compound of formula I and their pharmaceutically tolerable derivatives, solvates and stereoisomers for the preparation of a pharmaceutical for the treatment of Huntington's disease.

Preferably, the invention relates to the use of a compound selected from
2-amino-7-chloro-6-hydroxy-4-methyl-benzothiazole,
2-amino-4-chloro-6-hydroxy-7-methyl-benzothiazole,
2-amino-6-hydroxy-4-methyl-benzothiazole,
2-amino-5,7-dimethyl-6-hydroxy-benzothiazole,
2-dimethylamino-6-hydroxy-benzothiazole,
2-amino-4,7-dimethyl-6-hydroxy-benzothiazole,
6-methoxy-4,7-dimethyl-benzothiazol-2-ylamine,
N-(6-methoxy-4,7-dimethyl-benzothiazol-2-yl)-N-methylamine
and their salts, solvates and stereoisomers for the preparation of a pharmaceutical for the treatment of Huntington's disease.

Moreover, the invention relates to the use of a compound of formula I and their salts, solvates and stereoisomers for the preparation of a pharmaceutical for the treatment of spinal and bulbar muscular atrophy, dentatorubal pallidoluysian atrophy, spinocerebellar ataxia type-1, -2, -3, -6 and -7, Alzheimer's disease, bovine spongiform encephalopathy, primary systemic amyloidosis, secondary systemic amyloidosis, senile systemic amyloidosis, familial amyloid polyneuropathy I, hereditary cerebral amyloid angiopathy, hemodialysis-related amyloidosis, familial amyloid polyneuropathy III, Finnish hereditary systemic amyloidosis, type II diabetis, medullary carcinoma of thyroid, spongiform encephalopathies (prion diseases): Kuru, Gerstmann-Sträussler-Scheinker syndrome, familial insomnia, scrapie, atrial amyloidosis, hereditary non-neuropathic systemic amyloidosis, injection-localized amyloidosis, hereditary renal amyloidosis, amyotrophic lateral sclerosis, schizophrenia, sickle cell anaemia, unstable haemoglobin inclusion body haemolysis, α1-antitrypsin deficiency, antithrombin deficiency, thromboembolic disease and Parkinson's disease.

Moreover, the invention relates to the use of a compound selected from the
2-amino-7-chloro-6-hydroxy-4-methyl-benzothiazole,
2-amino-4-chloro-6-hydroxy-7-methyl-benzothiazole,
2-amino-6-hydroxy-4-methyl-benzothiazole,
2-amino-5,7-dimethyl-6-hydroxy-benzothiazole,
2-dimethylamino-6-hydroxy-benzothiazole,
2-amino-4,7-dimethyl-6-hydroxy-benzothiazole,
6-methoxy-4,7-dimethyl-benzothiazol-2-ylamine,
N-(6-methoxy-4,7-dimethyl-benzothiazol-2-yl)-N-methylamine
and their salts, solvates and stereoisomers for the preparation of a pharmaceutical for the treatment of spinal and bulbar muscular atrophy, dentatorubal pallidoluysian atrophy, spinocerebellar ataxia type-1, -2, -3, -6 and -7, Alzheimer's disease, bovine spongiform encephalopathy, primary systemic amyloidosis, secondary systemic amyloidosis, senile systemic amyloidosis, familial amyloid polyneuropathy I, hereditary cerebral amyloid angiopathy, hemodialysis-related amyloidosis, familial amyloid polyneuropathy III, Finnish hereditary systemic amyloidosis, type II diabetis, medullary carcinoma of thyroid, spongiform encephalopathies (prion diseases): Kuru, Gerstmann-amyloidosis, hereditary non-neuropathic systemic amyloidosis, injection-localized amyloidosis, hereditary renal amyloidosis, amyotrophic lateral sclerosis, schizophrenia, sickle cell anaemia, unstable haemoglobin inclusion body haemolysis, α1-antitrypsin deficiency, antithrombin deficiency, thromboembolic disease and Parkinson's disease.

Furthermore, the invention relates to the use of a compound selected from
*N*-(6-phenylcarbamoyl-benzothiazole-2-yl)-terephthalamic acid methyl ester,
3-methoxy-*N*-[4-(6-methyl-benzothiazole-2-yl)-phenyl]-benzamide,
3-amino-*N*-[4-(6-methyl-2,3-dihydro-benzothiazole-2-yl)-phenyl]-benzamide,
4-[(4-benzothiazole-2-yl-phenylimino)-methyl]-2,6-dibromo-benzene-1,3-diol,
benzothiazole-2,5,6-triamine,
[6,6']bibenzothiazolyl-2,2'-diamine,
6,6'-thiodi(benzothiazole-2-amine),
2,2'-*m*-phenylenedi(benzothiazole-6-amine),
and their salts, solvates and stereoisomers for the preparation of a pharmaceutical for inhibiting the formation of polyQ-aggregation.

Furthermore, the invention relates to the use of a compound selected from
*N*-(6-phenylcarbamoyl-benzothiazole-2-yl)-terephthalamic acid methyl ester,
3-methoxy-*N*-[4-(6-methyl-benzothiazole-2-yl)-phenyl]-benzamide,
3-amino-*N*-[4-(6-methyl-2,3-dihydro-benzothiazole-2-yl)-phenyl]-benzamide,
4-[(4-benzothiazole-2-yl-phenylimino)-methyl]-2,6-dibromo-benzene-1,3-diol,
benzothiazole-2,5,6-triamine,
[6,6']bibenzothiazolyl-2,2'-diamine,
6,6'-thiodi(benzothiazole-2-amine),
2,2'-*m*-phenylenedi(benzothiazole-6-amine),
and their salts, solvates and stereoisomers for the preparation of a pharmaceutical for the treatment of Huntington's disease.

Furthermore, the invention relates to compounds selected from the group consisting of
*N*-(6-phenylcarbamoyl-benzothiazol-2-yl)-terephthalamic acid methyl ester,
3-methoxy-*N*-[4-(6-methyl-benzothiazol-2-yl)-phenyl]-benzamide,
3-amino-*N*-[4-(6-methyl-2,3-dihydro-benzothiazol-2-yl)-phenyl]-benzamide,
4-[(4-benzothiazol-2-yl-phenylimino)-methyl]-2,6-dibromo-benzene-1,3-diol,
and their salts, solvates and stereoisomers.

Moreover, the invention relates to compounds selected from
4-{3-[4-(5-methyl-[1,2,4]oxadiazole-3-yl)-phenyl]-2-oxo-oxazolidine-5-ylmethyl}-piperazine-1-carboxylic acid (3,4-dichloro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-fluoro-3-nitro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-acetyl-phenyl)-amide,
1-{3-[4-(benzoylamino-imino-methyl)-phenyl]-2-oxo-oxazolidine-5-ylmethyl}-piperidine-4-carboxylic acid
and their salts, solvates and stereoisomers.

Furthermore, the invention relates to the use of a compound selected from the group consisting of
4-{3-[4-(5-methyl-[1,2,4]oxadiazole-3-yl)-phenyl]-2-oxo-oxazolidine-5-ylmethyl}-piperazine-1-carboxylic acid (3,4-dichloro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-fluoro-3-nitro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-acetyl-phenyl)-amide,
1-{3-[4-(benzoylamino-imino-methyl)-phenyl]-2-oxo-oxazolidine-5-ylmethyl}-piperidine-4-carboxylic acid
and their salts, solvates and stereoisomers for the preparation of a pharmaceutical for inhibiting the formation of polyQ-aggregation.

Furthermore, the invention relates to the use of a compound selected from
4-{3-[4-(5-methyl-[1,2,4]oxadiazole-3-yl)-phenyl]-2-oxo-oxazolidine-5-ylmethyl}-piperazine-1-carboxylic acid (3,4-dichloro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-fluoro-3-nitro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-acetyl-phenyl)-amide,
1-{3-[4-(benzoylamino-imino-methyl)-phenyl]-2-oxo-oxazolidine-5-ylmethyl}-piperidine-4-carboxylic acid
and their salts, solvates and stereoisomers for the preparation of a pharmaceutical for the treatment of Huntington's disease.

Moreover, the invention relates to compounds selected from
5-[4-(thiazole-2-ylcarbamoyl)-phenyl]-furane-2-carboxylic acid thiazole-2-yl-amide,
8-methoxy-1-methyl-1,2,3,4-tetrahydro-benzo[4,5]imidazo[1,2-*a*]pyrimidin-7-ylamine,
2,8,14,20-Tetrakis(2-chlorophenyl)-pentacyclo=[19.3.1.1^{3,7}.1^{9,13},1^{15,19}]octacosa-1 (25),3,5,7(28),9,11,13(27),15,17,19(26),21,23-dodecaen-4,6,10,12,16,18,22,24-octol,
5-[4-(2,4-dichloro-benzyloxy)-phenyl]-3*H*-[1,3,4]oxadiazole-2-thione
and their salts, solvates and stereoisomers.

Furthermore, the invention relates to the use of a compound selected from
5-[4-(thiazole-2-ylcarbamoyl)-phenyl]-furane-2-carboxylic acid thiazole-2-yl-amide,
8-methoxy-1-methyl-1,2,3,4-tetrahydro-benzo[4,5]imidazo-[1,2-*a*]pyrimidin-7-ylamine,
2,8,14,20-Tetrakis(2-chlorophenyl)-pentacyclo=[19.3.1.1^{3,7}.1^{9,13},1^{15,19}]octacosa-1(25),3,5,7(28),9,11,13(27),15,17,19(26),21,23-dodecaen-4,6,10,12,16,18,22,24-octol,
5-[4-(2,4-dichloro-benzyloxy)-phenyl]-3*H*-[1,3,4]oxadiazole-2-thione,
4-(6-methyl-benzooxazole-2-yl)-phenylamine,
2-(3-amino-phenyl)-quinoline-4-carboxylic acid,
2,7-dioxa-1,3,4,5,6,8,9,10-octaaza-dicyclopenta[*a,e*]cyclooctene
and their salts, solvates and stereoisomers for the preparation of a pharmaceutical for inhibiting the formation of polyQ-aggregation.

Furthermore, the invention relates to the use of a compound selected from
5-[4-(thiazole-2-ylcarbamoyl)-phenyl]-furane-2-carboxylic acid thiazole-2-yl-amide,
8-methoxy-1-methyl-1,2,3,4-tetrahydro-benzo[4,5]imidazo[1,2-*a*]pyrimidin-7-ylamine,
2,8,14,20-Tetrakis(2-chlorophenyl)pentacyclo=[19.3.1.1^{3,7}.1^{9,13},1^{15,19}]octacosa-1(25),3,5,7(28),9,11,13(27),15,17,19(26),21,23-dodecaen-4,6,10,12,16,18,22,24-octol,
5-[4-(2,4-dichloro-benzyloxy)-phenyl]-3*H*-[1,3,4]oxadiazole-2-thione,
4-(6-methyl-benzooxazole-2-yl)-phenylamine,
2-(3-amino-phenyt)-quinoline-4-carboxylic acid,
2,7-dioxa-3,3,4,5,6,8,9,10-octaaza-dicyclopenta[a,e]cyclooctene
and their salts, solvates and stereoisomers for the preparation of a pharmaceutical for the treatment of Huntington's disease.

The compounds mentioned-above are suitable as pharmaceutical active compounds for the treatment of Huntington's disease. They are furthermore suitable for the treatment of spinal and bulbar muscular atrophy, dentatorubal pallidoluysian atrophy, spinocerebellar ataxia type-1, -2, -3, -6 and -7, Alzheimer's disease, bovine spongiform encephalopathy, primary systemic amyloidosis, secondary systemic amyloidosis, senile systemic amyloidosis, familial amyloid polyneuropathy I, hereditary cerebral amyloid angiopathy, hemodialysis-related amyloidosis, familial amyloid polyneuropathy III, Finnish hereditary systemic amyloidosis, type II diabetis, medullary carcinoma of thyroid, spongiform encephalopathies (prion diseases): Kuru, Gerstmann- Straussler-Scheinker syndrome, familial insomnia, scrapie, atrial amyloidosis, hereditary non-neuropathic systemic amyloidosis, injection-localized amyloidosis, hereditary renal amyloidosis, amyotrophic lateral sclerosis, schizophrenia, sickle cell anaemia, unstable haemoglobin inclusion body haemolysis, α1-antitrypsin deficiency, antithrombin deficiency, thromboembolic disease and Parkinson's disease.

Finally they are suitable for the treatment of
Cystic fibrosis
Marfan syndrom
Amyotrophic lateral sclerosis
Scurvy
Maple syrup urine disease
Osteogenesis imperfecta
Cateracts
Familial hypercholesterolemia
α1-Antitrypsin deficiency
Tay-Sachs disease
Retinitis pigmentosa
Leprechaunism
Down's syndrome
Argyrophilic grain disease
Pick's disease
Corticobasal degeneration
Familial frontotemporal dementia
Non-Guamanian motor neurone disease
Niemann-Pick disease type C
Myotonic dystrophy
Hallervorden-Spatz disease.

For the identification of chemical compounds that prevent the formation of polyglutamine containing protein aggregates *in vitro* an automated filter retardation assay was developed. This assay is based on the finding that that polyglutamine-containing protein aggregates are insoluble in sodium dodecyl sulfate (SDS) and are retained on a cellulose acetate filter, whereas monomeric forms of the HD exon 1 protein with a polyglutamine sequence in the pathological range do not bind to the filter membrane.

The captured aggregates are then detected by simple immunoblot analysis using specific antibodies. The use of the filter retardation assay for the identification of chemical compounds that prevent the formation of huntingtin protein aggregates has been described (Scherzinger et al., 1997; Scherzinger et al., 1999; Wanker et al., 1999; Heiser et al., 2000; Wanker et al., 1998a; Wanker et al., 1998b).

For the evaluation of chemical compounds that have been identified by the high throughput screening a cell culture model system of HD has been developed. In this model system expression of HD exon 1 protein with a polyglutamine sequence in the pathological range (51 and 83 glutamines) is achieved through a tetracycline (tet)-regulated transactivator, a fusion protein consisting of the tet-repressor and a VP16 activation domain. This hybrid protein binds specifically to a tetracycline responsive DNA element TRE and promotes transcription from the adjacent CMV promoter. Tetracycine and its analogues such as doxycycline can bind to the transactivator and thereby prevent the hybrid protein from binding the TRE element. Thus, if doxycycline is present in the culture medium, transcription of mutant HD exon 1 protein is inhibited, while in its absence expression of HD exon 1 protein is induced. Formation and detection of SDS-insoluble huntingtin protein aggregates in this tetracycline-inducibe cell culture model system of HD has been described (Wälter et al., 2001).

### Literature:

Heiser, V., Scherzinger, E., Boeddrich, A., Nordhoff, E., Lurz, R., Schugardt, N., Lehrach, H., and Wanker, E. E. (2000). Inhibition of huntingtin fibrillogenesis by specific antibodies and small molecules: Implications for Huntington's disease therapy, Proc Natl Acad Sci U S A 97, 6739-6744.
Scherzinger, E., Lurz, R., Turmaine, M., Mangiarini, L., Hollenbach, B , Hasenbank, R., Bates, G. P., Davies, S. W., Lehrach, H., and Wanker, E. E. (1997). Huntingtin-encoded polyglutamine expansions form amyloid-like protein aggregates in vitro and in vivo, Cell 90, 549-58.
Scherzinger, E., Sittler, A., Schweiger, K., Heiser, V., Lurz, R., Hasenbank, R., Bates, G. P., Lehrach, H., and Wanker, E. E. (1999). Self-assembly of polyglutamine-containing huntingtin fragments into amyloid-like fibrils: implications for Huntington's disease pathology, Proc Natl Acad Sci U S A 96, 4604-9.
Wälter, S., Böddrich, A., Lurz, R., Scherzinger, E., Lüder, G., Lehrach, H., and Wanker, E. E. (2001). Accumulation of mutant huntingtin fragments in aggresome-like inclusion bodies as a result of insufficient protein degradation, Molecular Biology of the Cell, 12, 1393-1407 (2001).
Wanker, E. E., Scherzinger, E., Bates, G. P., and Lehrach, H. (1998a). Novel composition and method for the detection of diseases associated with amylid-like fibril or protein aggregate formation. In PCT/EP98/04811. Wanker, E. E., Scherzinger, E., Bates, G. P., and Lehrach, H. (1998b). Novel method of detecting amyloid-like fibrils or protein aggregates. In PCT/EP98/04810.
Wanker, E. E., Scherzinger, E., Heiser, V., Sittler, A., Eickhoff, H., and Lehrach, H. (1999). Membrane filter assay for detection of amyloid-like polyglutamine- containing protein aggregates, Methods Enzymol 309, 375-86.

Hydrates and solvates are understood as meaning, for example, the hemi-, mono- or dihydrates, solvates are understood as meaning, for example, alcohol addition compounds such as, for example, with methanol or ethanol.

The invention also relates to mixtures of the compounds of the formula I according to the invention, for example mixtures of two diastereomers, for example in the ratio 1:1, 1:2, 1:3, 1:4, 1:5, 1:10, 1:100 or 1:1000.
These are particularly preferably mixtures of stereoisomeric compounds.

For all radicals which occur more than once, such as, for example, A, their meanings are independent of one another.

A is alkyl, is unbranched (linear) or branched, and has 1, 2, 3, 4, 5 or 6 carbon atoms. A is preferably methyl, furthermore ethyl, propyl, isopropyl, butyl, isobutyl, sec-butyl or tert-butyl, furthermore also pentyl, 1-, 2- or 3-methylbutyl, 1,1- , 1,2- or 2,2-dimethylpropyl, 1-ethylpropyl, hexyl, furthermore preferably, for example, trifluoromethyl, pentafluoroethyl or 1,1,1-trifluoroethyl.

The compounds of the present invention and also the starting substances for their preparation are otherwise prepared by methods known per se, such as are described in the literature (e.g. in the standard works such as Houben-Weyl, Methoden der organischen Chemie [Methods of Organic Chemistry], Georg-Thieme-Verlag, Stuttgart), namely under reaction conditions which are known and suitable for the reactions mentioned. Use can also be made in this case of variants which are known per se, but not mentioned here in greater detail.

Synthesis of 2-amino-6-hydroxy-benzothiazoles is described by P.T.S. Lau and T.E. Gompf in J. Org. Chem. Vol. 35, 4103 - 4108.

It was found that under reactions conditions described in J. Org. Chem. (concentrated HCl) chlorinated side products are formed which can be separated from e.g. 2-amino-6-hydroxy-4-methyl-benzothiazole only with difficulties.

Surprisingly, by use of other strong acids like methanesulfonic acid, trifluoro acetic acid or formic acid, chlorination, or more generally halogenation if other halogen hydrogen acids are used, is avoided.

Benzothiazoles can also be prepared from anilines via thioureas (obtained according to C.R. Rasmussen Synthesis 1988,456 or Organic Synthesis, volume III, 735 (1955)) and subsequent treatment with sulfinylchloride according to the procedure of Th. Papenfuhs (Angewandte Chemie 94, 544 (1982).

Suitable inert solvents are, for example, hydrocarbons such as hexane, petroleum ether, benzene, toluene or xylene; chlorinated hydrocarbons such as trichloroethylene, 1,2-dichloroethane, carbon tetrachloride, chloroform or dichloromethane; alcohols such as methanol, ethanol, isopropanol, n-propanol, n-butanol or tert-butanol; ethers such as diethyl ether, diisopropyl ether, tetrahydrofuran (THF) or dioxane; glycol ethers such as ethylene glycol monomethyl or monoethyl ether (methyl glycol or ethyl glycol), ethylene glycol dimethyl ether (diglyme); ketones such as acetone or butanone; amides such as acetamide, dimethylacetamide, N-methylpyrrolidone (NMP) or dimethylformamide (DMF); nitriles such as acetonitrile; sulfoxides such as dimethyl sulfoxide (DMSO); carbon disulfide; carboxylic acids such as formic acid or acetic acid; nitro compounds such as nitromethane or nitrobenzene; esters such as ethyl acetate or mixtures of the solvents mentioned.

A base can be converted with an acid into the associated acid addition salt, for example by reaction of equivalent amounts of the base and of the acid in an inert solvent such as ethanol and subsequent evaporation. Suitable acids for this reaction are in particular those which yield physiologically acceptable salts. Thus inorganic acids can be used, e.g. sulfuric acid, nitric acid, halohydric acids such as hydrochloric acid or hydrobromic acid, phosphoric acids such as orthophosphoric acid, sulfamic acid, furthermore organic acids, in particular aliphatic, alicyclic, araliphatic, aromatic or heterocyclic mono- or polybasic carboxylic, sulfonic or sulfuric acids, e.g. formic acid, acetic acid, propionic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, p-toluenesulfonic acid, naphthalenemono- and -disulfonic acids and laurylsulfuric acid. Salts with physiologically unacceptable acids, e.g. picrates, can be used for the isolation and/or purification of the compounds of the formula I.

On the other hand, compounds can be converted into the corresponding metal salts, in particular alkali metal salts or alkaline earth metal salts, or into the corresponding ammonium salts using bases (e.g. sodium or potassium hydroxide or carbonate).
Physiologically acceptable organic bases, such as, for example, ethanolamine, can also be used.

The invention furthermore relates to pharmaceutical preparations comprising at least a compound selected from the group
2-amino-6-hydroxy-4,7-dimethyl-benzothiazole hydrochloride,
2-amino-6-hydroxy-4,7-dimethyl-benzothiazole methanesulfonate hydrate,
2-amino-7-chlor-6-hydroxy-4-methyl-benzothiazole,
6-methoxy-4,7-dimethyl-benzothiazol-2-ylamine or
N-(6-methoxy-4,7-dimethyl-benzothiazol-2-yl)-N-methylamine.

These preparations can be used as medicaments in human or veterinary medicine. Possible vehicles are organic or inorganic substances which are suitable for enteral (e.g. oral) or parenteral administration or topical application and do not react with the novel compounds, for example water, vegetable oils, benzyl alcohols, alkylene glycols, polyethylene glycols, glycerol triacetate, gelatin, carbohydrates such as lactose or starch, magnesium stearate, talc and petroleum jelly. In particular, tablets, pills, coated tablets, capsules, powders, granules, syrups, juices or drops are used for oral administration, suppositories are used for rectal administration, solutions, preferably oily or aqueous solutions, furthermore suspensions, emulsions or implants, are used for parenteral administration and ointments, creams or powders are used for topical application, or transdermally in patches.
The novel compounds can also be lyophilized and the lyophilizates obtained can be used, for example, for the production of injection preparations. The preparations indicated can be sterilized and/or can contain excipients such as lubricants, preservatives, stabilizers and/or wetting agents, emulsifiers, salts for influencing the osmotic pressure, buffer substances, colourants, flavourings and/or one or more further active compounds, e.g. one or more vitamins.
Pharmaceutical preparations which are suitable for administration in the form of aerosols or sprays are, for example, solutions, suspensions or emulsions of the active compound in a pharmaceutically acceptable solvent.

The compounds of the present invention and their physiologically acceptable salts and solvates can be used for the treatment and/or prophylaxis of the diseases or disease conditions indicated above.

In this context, the substances according to the invention are as a rule preferably administered in doses between approximately 0.1 and 100 mg, in particular between 1 and 10 mg, per dose unit. The daily dose is preferably between approximately 0.001 and 10 mg/kg of body weight. The specific dose for each patient, however, depends on all sorts of factors, for example on the efficacy of the specific compound employed, on the age, body weight, general state of health, sex, on the diet, on the time and route of administration, on the excretion rate, pharmaceutical combination and severity of the particular disorder to which the therapy applies. Oral administration is preferred.

Above and below, all temperatures are indicated in °C. In the following examples, "customary working up" means: if appropriate, water is added, the mixture is adjusted, if necessary, depending on the constitution of the final product, to a pH of between 2 and 10 and extracted with ethyl acetate or dichloromethane, the organic phase is separated off, dried over sodium sulfate and evaporated, and the residue is purified by chromatography on silica gel and/or by crystallization.

| | |
|---|---|
| Mass spectrometry (MS): | EI (electron impact ionization) M⁺ |
| | FAB (fast atom bombardment) (M+H)⁺ |

### Example 1

1.7 ml methanesulfonic acid is added to 1.4 g thiourea in 30 ml methanol, 5.0 g 2,5-dimethyl-1,4-benzoquinone in 110 ml hot methanol is added and the mixture is stirred at room temperature for 5 days.
The mixture is filtered, the solvent is removed and the residue is washed with acetone.
5.3 g 2-amino-4,7-dimethyl-6-hydroxy-benzothiazole, methanesulfonate hydrate is obtained, m.p. 199-201°, from 2,5-dimethyl-1,4-benzoquinone.

### Example 2:

14 g 2-Methyl-5-chloroaniline is treated with ammonium isothiocyanate to obtain the N-(2-methyl-5-chlorophenyl)-thiourea that is subsequently treated with sulfinylchloride at 50°C. The reaction is treated with excess water, stirred under heating for 30 min and filtered. The filtrate is treated with ammonia to reach pH 8. The product precipitated and is filtered off to yield 15 g 2-Amino-7-chloro-4-methylbenzothiazole mp. 206°C.

### Example 3:

1.5 g 2-amino-4,7-dimethyl-6-hydroxy-benzothiazole is dissolved in 20 ml acetonitril, 2 g potassium carbonate is added and at room temperature treated with 1.5 ml methyl iodide. After stirring at 40° for 3 hours, the reaction mixture is treted with water and extracted with ethyl acetate. The organic layer is separated, dried and evaporated. After chromatography with silica gel, 1.05 g 6-methoxy-4,7-dimethyl-benzothiazol-2-ylamine, m.p. 225-228°, and 50 mg N-(6-methoxy-4,7-dimethyl-benzothiazol-2-yl)-N-methylamine, m.p. 180-182° are isolated.

### Pharmacological Tests

### Testsystems:

In vitro: Proteolytic cleavage of GST-Huntington fusion-protein. Quantification of the precipitated aggregates after 18 h (filter retardation assay. Protein conc. ca. 0.65 µM).

In vivo: Incubation of the stable celt-line Tet-off (10 µM, 72 h). Lysates are used for quantification of aggregates and determination of the overall protein amount.

The following compounds
2-amino-4-methyl-6-hydroxy-benzothiazole (EMD 59966),
2-amino-4,7-dimethyl-6-hydroxy-benzothiazole
methanesulfonate hydrate (EMD 393607),
2-amino-4,7-dimethyl-6-hydroxy-benzothiazole hydrochloride (EMD 391979),
have been tested in comparison to 2-amino-4-methyl-benzothiazole (EMD 390908), which is known from EP 282971.

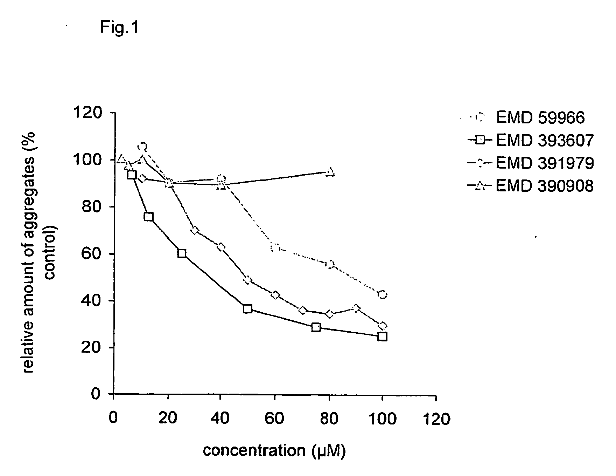

Compounds (EMD 59966), (EMD 393607) and (EMD 391979) show a significant decrease of the formation of polyQ-aggregation (Fig. 1).

The following examples relate to pharmaceutical preparations:

### Example A: Injection vials

A solution of 100 g of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole and 5 g of disodium hydrogenphosphate is adjusted to pH 6.5 using 2N hydrochloric acid in 3 l of double-distilled water, sterile filtered, dispensed into injection vials, lyophilized under sterile conditions and aseptically sealed. Each injection vial contains 5 mg of active compound.

### Example B: Suppositories

A mixture of 20 g of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole is fused with 100 g of soya lecithin and 1400 g of cocoa butter, poured into moulds and allowed to cool. Each suppository contains 20 mg of active compound.

### Example C: Solution

A solution is prepared from 1 g 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole, 9.38 g of NaH₂PO₄ · 2 H₂O, 28.48 g of Na₂HPO₄ · 12 H₂O and 0.1 g of benzalkonium chloride in 940 ml of doubled-distilled water. It is adjusted to pH 6.8, made up to 1 l and sterilized by irradiation. This solution can be used in the form of eye drops.

### Example D: Ointment

500 mg of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole are mixed with 99.5 g of petroleum jelly under aseptic conditions.

### Example E: Tablets

A mixture of 1 kg of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole, 4 kg of lactose, 1.2 kg of potato starch, 0.2 kg of talc and 0.1 kg of magnesium stearate is compressed in a customary manner to give tablets such that each tablet contains 10 mg of active compound.

### Example F: Coated tablets

Tablets are pressed analogously to Example E and then coated in a customary manner with a coating of sucrose, potato starch, talc, tragacanth and colourant.

### Example G: Capsules

2 kg of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole are filled into hard gelatin capsules in a customary manner such that each capsule contains 20 mg of the active compound.

### Example H: Ampoules

A solution of 2-amino-6-hydroxy-4,7-dimethyl-benzothiazole hydrochloride, 2-amino-6-hydroxy-4,7-dimethyl-benzothiazole methanesulfonate hydrate or 2-amino-7-chlor-6-hydroxy-4-methyl-benzothiazole in 60 l of double-distilled water is sterile filtered, dispensed into ampoules, lyophilized under sterile conditions and aseptically sealed. Each ampoule contains 10 mg of active compound.

## Claims

1. Use of a compound of formula I 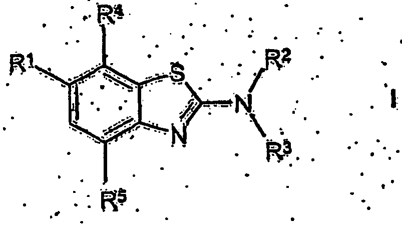 wherein
R¹ is OH, OA or Hal
R², R³ are independently of each other H or A,
R² and R³ together are an alkylene chain with 4, 5 or 6 C atoms,
R⁴, R⁵ are independently of each other A or Hal,
A is alkyl with 1, 2, 3, 4, 5 or 6 C atoms,
Hal is F, CI, Br or I,
and their salts, solvates and stereoisomers for the preparation of a pharmaceutical for the treatment of Huntington's disease, spinal or bulbar muscular atrophy, dentatorubal pallidoluysian atrophy, spinocerebellar ataxia type-1, -2, -3, -6 and -7, Alzheimer's disease, bovine spongiform encephalopathy, primary systemic amyloidosis, secondary systemic amyloidosis, senile systemic amyloidosis, familial amyloid polyneuropathy I, hereditary cerebral amyloid angiopathy, hemodialysis-related amyloidosis, familial amyloid polyneuropathy III, Finnish hereditary systemic amyloidosis, type II diabetis, medullary carcinoma of thyroid, spongiform encephalopathies (prion diseases), Kuru, Gerstmann-Sträussler-Scheinker syndrome, familial insomnia, scrapie, atrial amyloidosis, hereditary non-neuropathic systemic amyloidosis, amyotrophic lateral sclerosis, schizophrenia, sickle cell anaemia, unstable haemoglobin inclusion body haemolysis, α1-antitrypsin deficiency, antithrombin deficiency, thromboembolic disease, Parkinson's disease, Cystic fibrosis, Marfan syndrome, Scurvy, Maple syrup urine disease, Osteogenesis imperfecta, Cateracts, Familial hypercholesterolemia, Tay-Sachs disease, Retinitis pigmentosa, Leprechaunism, Down's syndrome, Argyrophilic grain disease, Pick's disease, Corticobasal degeneration, Familial frontotemporal dementia, Non-Guamanian motor neurone disease, Niemann-Pick disease type C, Myotonic dystrophy, Hallervorden-Spatz disease.

2. The use according to claim 1, wherein said compound is selected from
2-amino-7-chloro-6-hydroxy-4-methyl-benzothiazole,
2-amino-4-chloro-6-hydroxy-7-methyl-benzothiazole,
2-amino-4,7-dimethyl-6-hydroxy-benzothiazole,
6-methoxy-4,7-dimethyl-benzothiazol-2-ylamine,
N-(6-methoxy-4,7-dimethyl-benzothiazol-2-yl)-N-methylamine
and their salts, solvates and stereoisomers.

3. Benzothiazole derivatives selected from
2-amino-7-chloro-6-hydroxy-4-methyl-benzothiazole,
2-amino-4-chloro-6-hydroxy-7-methyl-benzothiazole,
6-methoxy-4,7-dimethyl-benzothiazol-2-ylamine,
N-(6-methoxy-4,7-dimethyl-benzothiazol-2-y)-N-methylamine,
N-(6-phenylcarbamoyl-benzothiazol-2-yl)-terephthalamic acid methyl ester,
3-methoxy-N-[4-(6-methyl-benzothiazol-2-yl)-phenyl]-benzamide,
3-amino-N-[4-(6-methyl-2,3-dihydro-benzothiazol-2-yi)-phenyl]-benzamide,
4-[(4-benzothiazol-2-yl-phenylimino)-methyl]-2,6-dibromobenzene-1,3-diol,
and their salts, solvates and stereoisomers.

4. The compound 2-amino-4,7-dimethyl-6-hydroxy-benzothiazole methanesulfonate hydrate.

5. Pharmaceutical preparation comprising at least one compound selected from
2-amino-6-hydroxy-4,7-dimethyl-benzothiazole hydrochloride,
2-amino-6-hydroxy-4,7-dimethyl-benzothiazole methanesulfonate hydrate, .
2-amino-7-chloro-6-hydroxy-4-methyl-benzothiazole,
6-methoxy-4,7-dimethyl-benzothiazol-2-ylamine, or
N-(6-methoxy-4,7-dimethyl-benzothiazol-2-yl)-N-methylamine.

6. Use of a compound selected from
N-(6-phenylcarbamoyl-benzothiazole-2-yl)-terephthalamic acid methyl ester,
3-amino-N-[4-(6-methyl-benzothiazole-2-yl)-phenyl]-benzamide,
3-amino-N-[4-(6-methyl-2,3-dihydro-benzothiazole-2-yl)-phenyl]-benzamide,
4-[(4-benzothiazole-2-yl-phenylimino)-methyl]-2,6-dibromo-benzene-1,3-diol,
benzothiazole-2,5,6-triamine,
[6,6']dibenzothiazolyl-2,2'-diamine,
6,6'-thiodi(benzothiazole-2-amine),
2,2'-m-phenylenedi(benzothiazole-6-amine),
4-{3-[4-(5-methyl-[1,2,4]oxadiazole-3-yl)-phenyl]-2-oxo-oxazolidine-5-ylmethyl}-piperazine-1-carboxylic acid (3,4-dichloro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-fluoro-3-nitro-phenyl)-amide,
4-{3-[4-(5-methyl-[1,2,4]oxadiazole-3-yl)phenyl]-2-oxo-oxazolidine-5-ylmethyl}-piperazine-1-carboxylic acid (4-acetyl-phenyl)amide,
1-{3-[4-(benzoylamino-imino-methyl)-phenyl]-2-oxo-oxazolidine-5-ylmethyl}-piperidine-4-carboxylic acid,
5-[4-(thiazole-2-ylcarbamoyl)-phenyl]-furane-2-carboxylic acid thiazole-2-yl-amide,
8-methoxy-1-methyl-1,2,3,4-tetrahydro-benzo[4,5]imidazo-[1,2-a]pyrimidin-7-ylamine,
2,8,14,20-Tetrakis(2-chlorophenyl)pentacyclo=[19.3.1.1^{3,7}.1^{9,13},1^{15,19}]octacosa-(25), 3, 5, 7(28), 9, 11, 13(27), 15, 17, 19(26), 21, 23-dodecaen-4, 6, 10, 12, 16, 18, 22, 24-octol,
5-[4-(2,4-dichloro-benzylox)yphenyl]-3*H*-[1,3,4]oxadiazole-2-thione,
4-(6-methyl-benzooxazole-2-yl)-phenylamine,
2-(3-amino-phenyl)-quinoline-4-carboxylic acid,
2,7-dioxa-1,3,4,5,6,8,9,10-octaaza-dicyclopenta[a,e]cyclooctene
and their salts, solvates and stereoisomers for the preparation of a pharmaceutical for the treatment of Huntington's disease, spinal or bulbar muscular atrophy, dentatorubal pallidoluysian atrophy, spinocerebellar ataxia type-1, -2, -3, -6 and -7, Alzheimer's disease, bovine spongiform encephalopathy, primary systemic amyloidosis, secondary systemic amyloidosis, senile systemic amyloidosis, familial amyloid polyneuropathy I, hereditary cerebral amyloid angiopathy, hemodialysis-related amyloidosis, familial amyloid polyneuropathy III, Finnish hereditary systemic amyloidosis, type II diabetis, medullary carcinoma of thyroid, spongiform encephalopathies (prion diseases); Kuru, Gerstmann-Sträussler-Scheinker syndrome, familial insomnia, scrapie, atrial amyloidosis, hereditary non-neuropathic systemic amyloidosis, amyotrophic lateral sclerosis, schizophrenia, sickle cell anaemia, unstable haemoglobin inclusion body haemolysis, α1-antitrypsin deficiency, antithrombin deficiency, thromboembolic disease, Parkinson's disease, Cystic fibrosis, Marfan syndrome, Scurvy, Maple syrup urine disease, Osteogenesis imperfecta, Cateracts, Familial hypercholesterolemia, Tay-Sachs disease, Retinitis pigmentosa, Leprechaunism, Down's syndrome, Argyrophilic grain disease, Pick's disease, Corticobasal degeneration, Familial frontotemporal dementia, Non-Guamanian motor neurone disease, Niemann-Pick disease type C, Myotonic dystrophy, Hallervorden-Spatz disease.

7. Compounds selected from
4-{3-[4-(5-methyl-[1,2,4]oxadiazole-3-yl)-phenyl]-2-oxo-oxazolidine-5-ylmethyl}-piperazine-1-carboxylic acid (3,4-dichloro-phenyl)-amide,
4-{3-[4-(5-methyl-[1,2,4]oxadiazole-3-yl)-phenyl]-2-oxo-oxazolidinel-5-ylmethyl}-piperazine-1-carboxylic acid (4-fluoro-3-nitro-phenyl)-amide,
4-{3-[4-(5-methyl-[1,2,4]oxadiazole-3-yl)-phenyl]-2-oxo-oxazolidine-5-ylmethyl}-piperazine-1-carboxylic acid (4-acetyl-phenyl)-amide,
1-{3-[4-(benzoylamino-imino-methyl)-phenyl]-2-oxo-oxazolidine-5-ylmethyl}-piperidine-4-carboxylic acid,
5-[4-(thiazole-2-ylcarbamoyl)-phenyl]-furane-2-carboxylic acid thiazole-2-yl-amide,
8-methoxy-1-methyl-1,2,3,4-tetrahydro-benzo[4,5]imidazo-[1,2-a]pyrimidin-7-ylamine,
2,8,14,20-Tetrakis(2-chlorophenyl)-pentacyclo=[19.3.1.1^{3,7}.1^{9,13}.1,^{15,19}]octacosa-1(25), 3, 5, 7(28), 9.11,13(27), 15, 17, 19(26), 21, 23-dodecaen-4, 6, 10, 12, 16, 18,22, 24-octol,
5-[4-(2,4-dichloro-benzyloxy)-phenyl]-3*H*-[1,3,4]oxadiazole-2-thione.
and their salts, solvates and stereoisomers.

## Patentansprüche

1. Verwendung einer Verbindung der Formel 1 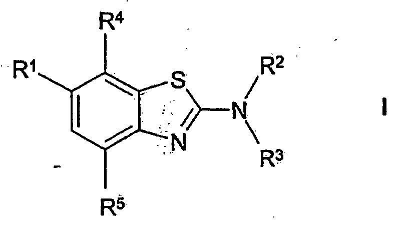 wobei:
R¹ OH, OA oder Hal ist
R², R³ unabhängig von einander H oder A sind,
R² und R³ zusammen eine Alkylenkette mit 4, 5 oder 6 C-Atomen sind,
R⁴, R⁵ unabhängig von einander A oder Hal sind,
A Alkyl mit 1, 2, 3, 4, 5 oder 6 C-Atomen ist,
Hal F, Cl, Br oder I ist,
und seiner Salze, Solvate und Stereoisomere zur Herstellung eines Arzneimittels zur Behandlung von Chorea Huntington, spinaler oder bulbärer Muskelatrophie, dentrorubaler pallidoluysischer Atrophie, spinozerebellärer Ataxie Typ 1, 2, 3, 6 und 7, Alzheimer' Krankheit, boviner spongioformer Encephalopathie, primärer systemischer Amyloidose, sekundärer systemischer Amyloidose, seniler systemischer Amyloidose, familiärer amyloider Polyneuropathie I, erblicher zerebraler amyloider Angiopathie, Hämodialyseverwandter Amyloidose, familiärer amyoloider Polyneuropathie III, finnischer erblicher systemischer Amyloidose, Typ-11 Diabetes, medullärem Karzinom der Schilddrüse, spongiformer Enzephalopathien (Prionenkrankheiten), Kuru, Gerstmann-Sträussler-Scheinker-Syndrom, familiärer Insomnie, Traberkrankheit, atrialer Amyloidose, erblicher nicht-neurophatischer systemischer Amyloidose, amyotropher Lateralsklerose, Schizophrenie, Sichelzellanämie, instabiler Hämoglobin-einschlusskörperhämolyse, α1-Antitrypsindefizienz, Antithrombindefizienz, thromboembolischer Krankheit, Parkinson'scher Krankheit, zystischer Fibrose, Marfansyndrom, amylotropher Lateralsklerose, Skorbut, Ahornsirup-Krankheit, Osteogenesis imperfecta, Katerakten, familiärer Hypercholesterinämie, Tay-Sachs-Krankheit, Retinitis pigmentosa, Leprechaunismus, Down-Syndrom, argyrophiler Körnerkrankheit, Pick's Krankheit, kortikobasaler Degeneration, familiärer frontotemporaler Demenz, nicht-guamanischer Motorneuronenerkrankung, Niemann-Pick Krankheit Typ C, myotonischer Dystrophie, Hallervorden-Spatz-Erkrankung.

2. Verwendung nach Anspruch 1, wobei die Verbindung ausgewählt ist aus:
2-Amino-7-chlor-6-hydroxy-4-methyl-benzothiazol,
2-Amino-4-chlor-6-hydroxy-7-methyl-benzothiazol,
2-Amino-4,7-dimethyl-6-hydroxy-benzothiazol,
6-Methoxy-4,7-dimethyl-benzothiazol-2-ylamin,
N-(6-Methoxy-4,7-dimethyl-benzothiazol-2-yl)-N-methylamin,
und ihren Salzen, Solvaten und Stereoisomeren.

3. Benzothiazolderivate ausgewählt aus:
2-Amino-7-chlor-6-hydroxy-4-methyl-benzothiazol,
2-Amino-4-chlor-6-hydroxy-7-methyl-benzothiazol,
6-Methoxy-4,7-dimethyl-benzothiazol-2-ylamin,
N-(6-Methoxy-4,7-dimethyl-benzothiazol-2-yl)-N-methylamin,
N-(6-Phenylcarbamoyl-benzothiazol-2-yl)-terephthalamsäuremethylester,
3-Methoxy-N-[4-(6-methyl-benzothiazol-2-yl)-phenyl]-benzamid,
3-Amino-N-[4-(6-methyl-2,3-dihydro-benzothiazol-2-yl)-phenyl]-benzamid,
4-[(4-Benzothiazol-2-yl-phenylimino)-methyl]-2,6-dibrombenzen-1,3-diol,
und ihren Salzen, Solvaten und Stereoisomeren.

4. Verbindung 2-Amino-4,7-dimethyl-6-hydroxy-benzothiazolmethansulfonathydrat.

5. Pharmazeutische Zusammensetzung, umfassend mindestens eine Verbindung ausgewählt aus:
2-Amino-6-hydroxy-4,7-dimethyl-benzothiazolhydrochlorid,
2-Amino-6-hydroxy-4,7-dimethyl-benzothiazolmethansulfonathydrat,
2-Amino-7-chlor-6-hydroxy-4-methyl-benzothiazol,
6-Methoxy-4,7-dimethyl-benzothiazol-2-ylamin, oder
N-(6-Methoxy-4,7-dimethyl-benzothiazol-2-yl)-N-methylamin.

6. Verwendung einer Verbindung, ausgewählt aus:
N-(6-Phenylcarbamoyl-benzothiazol-2-yl)-terephthalamsäuremethylester,
3-Amino-N-[4-(6-methyl-benzothiazol-2-yl)-phenyl]-benzamid,
3-Amino-N-[4-(6-methyl-2,3-dihydro-benzothiazol-2-yl)-phenyl]-benzamid,
4-[(4-Benzothiazol-2-yl-phenylimino)-methyl]-2,6-dibrombenzen-1,3-diol,
Benzothiazol-2,5,6-triamin,
[6,6']Dibenzothiazolyl-2,2'-diamin,
6,6'-Thiodi(benzothiazol-2-amin),
2,2'-m-Phenylenedi(benzothiazol-6-amin),
4-{3-[4-(5-Methyl-[1,2,4]oxadiazol-3-yl)-phenyl]-2-oxo-oxazolidin-5-ylmethyl}-piperazin-1-carbonsäure-(3,4-dichlor-phenyl)-amid,
4-{3-[4-(5-Methyl-[1,2,4]oxadiazol-3-yl)-phenyl]-2-oxo-oxazolidin-5-ylmethyl}-piperazin-1-carbonsäure-(4-fluor-3-nitro-phenyl)-amid,
4-{3-[4-(5-Methyl-[1,2,4]oxadiazol-3-yl)-phenyl]-2-oxo-oxazolidin-5-ylmethyl}-piperazin-1-carbonsäure-(4-acetyl-phenyl)-amid,
1-{3-[4-(Benzoylamino-imino-methyl)-phenyl]-2-oxo-oxazolidin-5-ylmethyl}-piperidin-4-carbonsäure,
5-[4-(Thiazol-2-ylcarbamoyl)-phenyl]-furan-2-carbonsäurethiazol-2-yl-amid,
8-Methoxy-1-methyl-1,2,3,4-tetrahydro-benzo[4,5]imidazo-[1,2-a]pyrimidin-7-ylamin,
2,8,14,20-Tetrakis(2-chlorphenyl)-pentacyclo=[19.3.1.1^{3,7}.1^{9,13},1^{15,19}]octacosa-(25), 3, 5, 7(28), 9, 11, 13(27), 15, 17, 19(26), 21, 23-dodecaen-4, 6, 10, 12, 16, 18, 22, 24-octol,
5-[4-(2,4-Dichloro-benzyloxy)-phenyl]-3H [1,3,4]oxadiazol-2-thion,
4-(6-Methyl-benzooxazol-2-yl)-phenylamin,
2-(3-Amino-phenyl)-chinoline-4-carbonsäure,
2,7-Dioxa-1,3,4,5,6,8,9,10-octaaza-dicyclopenta[a,e]cycloocten
und seiner Salze, Solvate und Stereoisomere zur Herstellung eines Arzneimittels zur Behandlung von Chorea Huntington, spinaler oder bulbärer Muskelatrophie, dentrorubaler pallidoluysischer Atrophie, spinozerebellärer Ataxie Typ 1, 2, 3, 6 und 7, Alzheimer' Krankheit, boviner spongioformer Encephalopathie, primärer systemischer Amyloidose, sekundärer systemischer Amyloidose, seniler systemischer Amyloidose, familiärer amyloider Polyneuropathie I, erblicher zerebraler amyolider Angiopathie, Hämodialysebezogener Amyloidose, familiärer amyoloider Polyneuropathie III, finnischer erblicher systemischer Amyloidose, Typ-II Diabetes, medullärem Karzinom der Schilddrüse, spongiformer Enzephalopathien (Prionenkrankheiten), Kuru, Gerstmann-Sträussler-Scheinker-Syndrom, familiärer Insomnie, Traberkrankheit, atrialer Amyloidose, erblicher nicht-neurophatischer systemischer Amyloidose, amyotropher Lateralsklerose, Schizophrenie, Sichelzellanämie, instabiler Hämoglobineinschlusskörperhämolyse, α1-Antitrypsindefizienz, Antithrombindefizienz, thromboembolischer Krankheit, Parkinson'scher Krankheit, zystischer Fibrose, Marfansyndrom, amylotropher Lateralsklerose, Skorbut, Ahornsirup-Krankheit, Osteogenesis imperfecta, Katerakten, familiärer Hypercholesterinämie, Tay-Sachs-Krankheit, Retinitis pigmentosa, Leprechaunismus, Down-Syndrom, argyrophiler Körnerkrankheit, Pick's Krankheit, kortikobasaler Degeneration, familiärer frontotemporaler Demenz, nicht-guamanischer Motorneuronenerkrankung, Niemann-Pick Krankheit Typ C, myotonischer Dystrophie, Hallervorden-Spatz-Erkrankung.

7. Verbindungen ausgewählt aus:
4-{3-[4-(5-Methyl-[1,2,4]oxadiazol-3-yl)-phenyl]-2-oxo-oxazolidin-5-ylmethyl}-piperazin-1-carbonsäure (3,4-dichlorphenyl)-amid,
4-{3-[4-(5-Methyl-[1,2,4]oxadiazol-3-yl)-phenyl]-2-oxo-oxazolidin-5-ylmethyl}-piperazin-1-carbonsäure-(4-fluor-3-nitro-phenyl)-amid,
4-{3-[4-(5-Methyl-[1,2,4]oxadiazol-3-yl)-phenyl]-2-oxo-oxazolidin-5-ylmethyl}-piperazin-1-carbonsäure-(4-acetyl-phenyl)-amid,
1-{3-[4-(Benzoylamino-imino-methyl)-phenyl]-2-oxo-oxazolidin-5-ylmethyl}-piperidin-4-carbonsäure,
5-[4-(Thiazol-2-ylcarbamoyl)-phenyl]-furan-2-carbonsäure-thiazol-2-yl-amid,
8-Methoxy-1-methyl-1,2,3,4-tetrahydro-benzo[4,5]imidazo-[1,2-a]pyrimidin-7-ylamin,
2,8,14,20-Tetrakis(2-chlorophenyl)-pentacyclo=[19.3.1.1^{3,7}.1^{9,13}.1^{15,19}]octacosa-1(25), 3, 5, 7(28), 9, 11, 13(27), 15, 17, 19(26), 21, 23-dodecaen-4, 6, 10, 12, 16, 18, 22, 24-octol,
5-[4-(2,4-Dichlor-benzyloxy)-phenyl]-3*H*[1,3,4]oxadiazol-2-thion,
und ihren Salzen, Solvaten und Stereoisomeren.

## Revendications

1. Utilisation d'un composé de formule 1 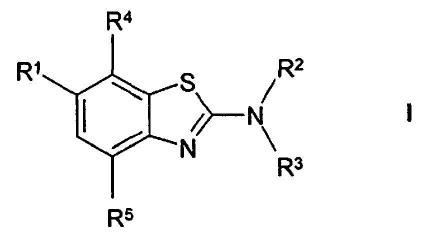 dans laquelle
R1 représente OH, OA ou Hal
R2, R3 représentent indépendamment l'un de l'autre H ou A,
R2 and R3 ensemble représentent une chaîne alkylène avec 4, 5 ou 6 atomes de C,
R4, R5 représentent indépendamment l'un de l'autre A ou Hal,
A représente un alkyle avec 1, 2, 3, 4, 5 ou 6 atomes de C,
Hal représente F, Cl, Br ou I,
et leurs sels, solvates et stéréoisomères pour la préparation d'un médicament pour le traitement de la maladie de Huntington, l'amyotrophie spinale ou bulbaire, l'atrophie dentatorubropallidoluysienne, l'ataxie spinocérébelleuse de type 1, 2, 3, 6 et 7, la maladie d'Alzheimer, l'encéphalopathie spongiforme bovine, amyloïdose systémique primaire, amyloïdose systémique secondaire, amyloïdose systémique sénile, polyneuropathie amyloïde familiale 1, angiopathie amyloïde cérébrale héréditaire, amyloïdose liée à une hémodialyse, polyneuropathie amyloïde familiale III, amyloïdose systémique héréditaire finlandaise, diabète de type II, carcinome médullaire de la thyroïde, encéphalopathies spongiformes (maladies à prions), Kuru, syndrome de Gerstmann-Stràussler-Scheinker, insomnie familiale, tremblante du mouton, amyloïdose atriale, amyloïdose systémique non neuropathique héréditaire, sclérose latérale amyotrophique, schizophrénie, drépanocytose, hémolyse de corps d'inclusion d'hémoglobine instable, déficience en α-1-antitrypsine, déficience en antithrombine, maladie thromboembolique, maladie de Parkinson, mucoviscidose, syndrome de Marfan, scorbut, leucinose, ostéogenèse imparfaite, cataractes, hypercholestérolémie familiale, maladie de Tay-Sachs, rétinite pigmentaire, lèprechaunisme, trisomie 21, maladie des grains argyrophiles, maladie de Pick, dégénérescence corticobasale, démence frontotemporale familiale, sclérose latérale amyotrophique non guamanienne, maladie de Niemann-Pick de type C, dystrophie myotonique, maladie de Hallervorden-Spatz.

2. Utilisation selon la revendication 1, dans laquelle le composé est choisi parmi
2-amino-7-chloro-6-hydroxy-4-méthyl-benzothiazole,
2-amino-4-chloro-6-hydroxy-7-méthyl-benzothiazole,
2-amino-4,7-diméthyl-6-hydroxy-benzothiazole,
6-méthoxy-4,7-diméthyl-benzothiazol-2-ylamine,
N-(6-méthoxy-4,7-diméthyl-benzothiazol-2-yl)-N-méthylamine
et leurs sels, solvates et stéréoisomères.

3. Dérivés de benzothiazole choisis parmi
2-amino-7-chloro-6-hydroxy-4-méthyl-benzothiazole,
2-amino-4-chloro-6-hydroxy-7-méthyl-benzothiazole,
6-méthoxy-4,7-diméthyl-benzothiazol-2-ylamine,
N-(6-méthoxy-4,7-diméthyl-benzothiazol-2-yl)-N-méthylamine,
ester méthylique de l'acide N-(6-phénylcarbamoyl-benzothiazol-2-yl)-téréphthalamique,
3-méthoxy-N-[4-(6-méthyl-benzothiazol-2-yl)-phényl]-benzamide,
3-amino-N-[4-(6-méthyl-2,3-dihydro-benzothiazol-2-yl)-phényl]-benzamide,
4-[(4-benzothiazol-2-yl-phénylimino)-méthyl]-2,6-dibromo-benzène-1,3-diol,
et leurs sels, solvates et stéréoisomères.

4. Composé hydrate de 2-amino-4,7-diméthyl-6-hydroxy-benzothiazole méthanesulfonate.

5. Préparation pharmaceutique comprenant au moins un composé choisi parmi
hydrochlorure de 2-amino-6-hydroxy-4,7-diméthyl-benzothiazole,
hydrate de 2-amino-6-hydroxy-4,7-diméthyl-benzothiazole méthanesulfonate,
2-amino-7-chloro-6-hydroxy-4-méthyl-benzothiazole,
6-méthoxy-4,7-diméthyl-benzothiazol-2-ylamine, ou
N-(6-méthoxy-4,7-diméthyl-benzothiazol-2-yl)-N-méthylamine.

6. Utilisation d'un composé choisi parmi
ester méthylique de l'acide N-(6-phénylcarbamoyl-benzothiazol-2-yl)-téréphathalamique,
3-amino-N-[4-(6-méthyl-benzothiazol-2-yl)-phényl]-benzamide,
3-amino-N-[4-(6-méthyl-2,3-dihydro-benzothiazol-2-yl)-phényl]-benzamide,
4-[(4-benzothiazol-2-yl-phénylimino)-méthyl]-2,6-dibromo-benzene-1,3-diol,
benzothiazole-2,5,6-triamine,
[6,6']dibenzothiazolyl-2,2'-diamine,
6,6'-thiodi(benzothiazole-2-amime),
2,2'-m-phénylenedi(benzothiazole-6-amine),
(3,4-dichloro-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
(4-fluoro-3-nitro-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
(4-acetyl-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
acide 1-{3-[4-(benzoylamino-imino-méthyl)-phényl]-2-oxo-oxazolidine-5-ylméthyl}-pipéridine-4-carboxylique,
thiazole-2-yl-amide de l'acide 5-[4-(thiazole-2-yl-carbamoyl)-phényl]-furane-2-carboxylique,
8-méthoxy-1-méthyl-1,2,3,4-tétrahydro-benzo[4,5]imidazo-[1,2-a]pyrimidin-7-ylamine,
2,8,14,20-Tetrakis(2-chlorophényl)-pentacyclo=[19.3.1.1^{3,7}.1^{9,13},1^{15,19}]octacosa-(25),3,5,7(28),9,11,13(27),15,17,19(26),21,23-dodecaèn-4,6,10,12,16,18,22,24-octol,
5-[4-(2,4-dichloro-benzyloxy)-phényl]-3H-[1,3,4]oxadiazole-2-thione,
4-(6-méthyl-benzooxazole-2-yl)-phénylamine,
acide 2-(3-amino-phényl)-quinoline-4-carboxylique,
2,7-dioxa-1,3,4,5,6,8,9,10-octaaza-dicyclopenta[a,e]cyclooctène
et leurs sels, solvates et stéréoisomères pour la préparation d'un médicament pour le traitement de la maladie de Huntington, l'amyotrophie spinale ou bulbaire, l'atrophie dentatorubropallidoluysienne, l'ataxie spinocérébelleuse de type 1, 2, 3, 6 et 7, la maladie d'Alzheimer, l'encéphalopathie spongiforme bovine, amyloïdose systémique primaire, amyloïdose systémique secondaire, amyloïdose systémique sénile, polyneuropathie amyloïde familiale 1, angiopathie amyloïde cérébrale héréditaire, amyloïdose liée à une hémodialyse, polyneuropathie amyloïde familiale III, amyloïdose systémique héréditaire finlandaise, diabète de type II, carcinome médullaire de la thyroïde, encéphalopathies spongiformes (maladies à prions), Kuru, syndrome de Gerstmann-Sträussler-Scheinker, insomnie familiale, tremblante du mouton, amyloïdose atriale, amyloïdose systémique non neuropathique héréditaire, sclérose latérale amyotrophique, schizophrénie, drépanocytose, hémolyse de corps d'inclusion d'hémoglobine instable, déficience en α-1-antitrypsine, déficience en antithrombine, maladie thromboembolique, maladie de Parkinson, mucoviscidose, syndrome de Marfan, scorbut, leucinose, ostéogenèse imparfaite, cataractes, hypercholestérolémie familiale, maladie de Tay-Sachs, rétinite pigmentaire, lèprechaunisme, trisomie 21, maladie des grains argyrophiles, maladie de Pick, dégénérescence corticobasale, démence frontotemporale familiale, sclérose latérale amyotrophique non guamanienne, maladie de Niemann-Pick de type C, dystrophie myotonique, maladie de Hallervorden-Spatz.

7. Composés choisis parmi
(3,4-dichloro-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
(4-fluoro-3-nitro-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
(4-acetyl-phényl)-amide de l'acide 4-{3-[4-(5-méthyl-[1,2,4]oxadiazole-3-yl)-phényl]-2-oxo-oxazolidine-5-ylméthyl}-pipérazine-1-carboxylique,
acide 1-{3-[4-(benzoylamino-imino-méthyl)-phényl]-2-oxo-oxazolidine-5-ylméthyl}-pipéridine-4-carboxylique,
thiazole-2-yl-amide de l'acide 5-[4-(thiazole-2-yl-carbamoyl)-phényl]-furane-2-carboxylique,
8-méthoxy-1-méthyl-1,2,3,4-tétrahydro-benzo[4,5]imidazo-[1,2-a]pyrimidin-7-ylamine,
2,8,14,20-Tetrakis(2-chlorophényl)-pentacyclo=[19.3.1.1^{3,7}.1^{9,13},1^{15,19}]octacosa-1(25),3,5,7(28),9,11,13(27),15,17,19(26),21,23-dodecaèn-4,6,10,12,16,18,22,24-octol,
5-[4-(2,4-dichloro-benzyloxy)-phényl]-3H-[1,3,4]oxadiazole-2-thione,
et leurs sels, solvates et stéréoisomères.
